(19) Europäisches Patentamt
European Patent Office
Office européen des brevets

(11) **EP 1 042 068 B1**

(12) **FASCICULE DE BREVET EUROPEEN**

(45) Date de publication et mention
de la délivrance du brevet:
**21.02.2007 Bulletin 2007/08**

(51) Int Cl.:
***B01J 25/04*** *(2006.01)*   ***C07C 209/48*** *(2006.01)*

(21) Numéro de dépôt: **98963616.2**

(86) Numéro de dépôt international:
**PCT/FR1998/002856**

(22) Date de dépôt: **23.12.1998**

(87) Numéro de publication internationale:
**WO 1999/033561 (08.07.1999 Gazette 1999/27)**

(54) **PROCEDE DE REGENERATION D'UN CATALYSEUR D'HYDROGENATION PROCEDE D'HYDROGENATION DE COMPOSES COMPRENANT DES FONCTIONS NITRILES**

REGENERIERUNGSVERFAHREN EINES HYDRIERUNGSKATALYSATOR UND VERFAHREN ZUR HYDRIERUNG VON NITRILFONKTIONELLEN VERBINDUNGEN

METHOD FOR REGENERATING A HYDROGENATION CATALYST, METHOD FOR HYDROGENATING COMPOUNDS COMPRISING NITRILE FUNCTIONS

(84) Etats contractants désignés:
**BE DE ES FR GB IT NL**

(30) Priorité: **29.12.1997 FR 9716832**

(43) Date de publication de la demande:
**11.10.2000 Bulletin 2000/41**

(73) Titulaire: **RHODIA POLYAMIDE INTERMEDIATES 69192 Saint-Fons (FR)**

(72) Inventeurs:
• **BOSCHAT, Vincent
F-69007 Lyon (FR)**
• **LECONTE, Philippe
F-69330 Meyzieu (FR)**

(74) Mandataire: **Esson, Jean-Pierre et al
Rhodia Services,
Direction de la Propriété Industrielle,
Crit-Carrières,
B.P. 62
69192 Saint-Fons Cédex (FR)**

(56) Documents cités:
**DE-C- 832 604        FR-A- 2 211 287
GB-A- 833 592        US-A- 2 604 455**

Remarques:
Le dossier contient des informations techniques présentées postérieurement au dépôt de la demande et ne figurant pas dans le présent fascicule.

**Description**

La présente invention concerne un procédé de régénération d'un catalyseur d'hydrogénation, et des procédés d'hydrogénation réalisés avec un catalyseur comprenant au moins le catalyseur régénéré.

Elle se rapporte plus particulièrement à un procédé de régénération des catalyseurs de type Raney utilisés dans les procédés d'hydrogénation totale ou partielle de composés comprenant des fonctions nitriles en fonctions amines.

L'hydrogénation des composés comprenant des fonctions nitriles en composés aminés est mise en oeuvre industriellement depuis très longtemps.

Ainsi, l'hexaméthylène diamine, un composé utilisé notamment pour la fabrication du polyhexaméthylène adipamide appelé également PA66 et connu sous le nom "Nylon", est fabriqué industriellement par hydrogénation de l'adiponitrile en présence d'un catalyseur Nickel de Raney. Ce procédé est notamment décrit dans le brevet US 3 821 305. D'autres procédés d'hydrogénation de composés nitriles ou polynitriles en composés aminés sont décrits dans les brevets US 3 372 195, US 2 287 219, US 2 449 036, US 3 565 957, US 3 998 881, US 4 186 146, US 4235 821. US 4 254 059, WO 95/17 959. US-A-2 604 455 décrit un procédé de regeneration de catalyseurs.

Ces documents se rapportent à l'hydrogénation de différents composés nitriles aliphatiques, aromatiques, substitués, insaturés ....

Ces brevets concernent également des procédés qui sont mis en oeuvre en présence de solvant, de soude et d'ammoniac. Ils sont généralement mis en oeuvre avec des catalyseurs type Raney tels que le nickel de Raney ou le cobalt de Raney.

La préparation de ces catalyseurs de Raney est décrite depuis très longtemps, et notamment dans le brevet US 1 638 190 et dans J.A.C.S. 54,4116 (1932). Un procédé de préparation à partir d'alliage nickel, molybdène, aluminium est décrit dans le brevet US 2 948 887.

Il a également été proposé des catalyseurs d'hydrogénation type Raney à effet catalytique amélioré par dopage avec d'autres éléments métalliques. Par exemple, le brevet US 4 153 578 décrit un catalyseur Nickel de Raney comprenant du molybdène. Ce catalyseur est notamment utilisé pour la réduction des aldéhydes en alcools.

Il a également été proposé des procédés d'hydrogénation de composés polynitriles par réduction de certaines fonctions nitriles pour produire des composés comprenant des fonctions nitriles et amines. Une application développée est l'hydrogénation partielle, appelée hémihydrogénation de dinitriles aliphatiques tels que l'adiponitrile en aminonitriles tels que l'aminocapronitrile. Ainsi, le brevet US 4 389 348 décrit l'hémihydrogénation de dinitrile en oméga-aminonitrile par l'hydrogène, en milieu solvant aprotique et ammoniac et en présence de rhodium déposé sur un support basique. Le brevet US 5 151 543 décrit l'hémihydrogénation de dinitriles en aminonitriles dans un solvant en excès molaire d'au moins 2/1 par rapport au dinitrile, comprenant l'ammoniac liquide ou un alcanol contenant une base minérale en présence d'un catalyseur type Nickel de Raney ou cobalt de Raney.

De même, la demande de brevet WO 93/16 034, décrit un procédé d'hémihydrogénation de l'adiponitrile en aminocapronitrile en présence de catalyseur Nickel de Raney d'une base et d'un complexe d'un métal de transition.

Dans les documents cités, le problème de la diminution de consommation de catalyseur soit par une meilleure récupération, soit par recyclage n'est pas envisagé.

La récupération du catalyseur d'hydrogénation est envisagée dans le cas de l'hydrogénation complète par le brevet US 4 429 159 qui décrit un procédé de prétraitement du catalyseur Nickel de Raney par un carbonate pour diminuer l'entraînement de celui-ci dans le flux d'hexaméthylène diamine. Le catalyseur ainsi récupéré peut être recyclé après lavage avec de l'eau, en mélange avec un catalyseur neuf.

Il a également été décrit des procédés de régénération des catalyseurs utilisés dans des procédés d'hémihydrogénation dans les demandes de brevets WO 97/37964 et WO 97/37963. Les catalyseurs sont traités par un flux d'hydrogène à une température comprise entre 150 et 400°C, en absence de tout liquide ou solvant. Les catalyseurs après régénération par traitement à l'hydrogène sont lavés par de l'eau jusqu'à pH neutre et éventuellement conditionnés par de l'ammoniac liquide. Ces procédés de régénération mettent en oeuvre un traitement à température élevée qui peut provoquer un frittage partiel du catalyseur. En outre, ces procédés ne permettent pas de récupérer une activité catalytique totale notamment quand le procédé d'hydrogénation des composés nitriles est réalisé en milieu liquide et plus particulièrement en présence d'un composé basique.

Un des buts de la présente invention est de proposer un procédé de régénération d'un catalyseur d'hydrogénation du type catalyseur de Raney permettant de récupérer une activité sensiblement équivalente à celle d'un catalyseur neuf.

A cet effet, l'invention propose un procédé de régénération d'un catalyseur de type catalyseur de Raney d'hydrogénation totale ou partielle des fonctions nitriles en fonctions amines de composés organiques caractérisé en ce qu'il consiste à mélanger le catalyseur usé séparé du milieu réactionnel d'hydrogénation, avec une solution aqueuse d'un composé basique présentant une concentration en anion supérieure à 0,01 mol/l, à maintenir le mélange à une température inférieure à 130°C, puis à laver le catalyseur traité par de l'eau ou une solution aqueuse basique jusqu'à un pH final des eaux de lavage compris entre 12 et 13.

Selon une autre caractéristique préférentielle de l'invention, le procédé de régénération du catalyseur peut comprendre une hydrogénation de celui-ci mis en oeuvre par traitement du catalyseur sous atmosphère d'hydrogène à une température inférieure à 130°C.

Selon l'invention, la mise sous atmosphère d'hydrogène du catalyseur peut être réalisée avant le maintien en température du mélange catalyseur usé/solution basique. Dans ce cas, le traitement basique et l'hydrogénation du catalyseur usé sont réalisés simultanément.

Dans un autre mode de réalisation, l'hydrogénation du catalyseur est réalisé avant l'étape de mélange avec la solution aqueuse basique. Enfin, il est également possible de soumettre à l'étape d'hydrogénation, le catalyseur traité par une solution basique et éventuellement lavé.

D'un point de vue économique et de simplicité de mise en oeuvre, le procédé consistant à réaliser simultanément le traitement par une solution basique et hydrogénation est préféré.

Les différentes caractéristiques et définitions des produits ou conditions opératoires décrites ci-après sont applicables à tous les modes de réalisation cités ci-dessus.

Le procédé de régénération de l'invention peut-être réalisé en discontinu ou en continu.

Le procédé de l'invention permet une régénération du catalyseur d'hydrogénation ou d'hémihydrogénation à basse température évitant ainsi une détérioration du catalyseur, plus particulièrement une diminution de l'effet dopant des éléments métalliques contenus dans le catalyseur de Raney.

Selon l'invention, les catalyseurs pouvant être régénérés par le procédé décrit ci-dessus sont les catalyseurs type Raney tel que, par exemple le Nickel de Raney, le cobalt de Raney. Ces catalyseurs peuvent comprendre avantageusement un ou plusieurs autres éléments, souvent appelés dopants, tels que par exemple le chrome, le titane, le molybdène, le tungstène, le manganèse, le vanadium, le zirconium, le fer, le zinc et plus généralement les éléments des groupes IIB, IVB, IIIB, VB, VIB, VIIB, et VIII de la table périodique de Mendeleev. Parmi ces éléments dopants, le chrome, le fer et/ou le titane, ou un mélange de ces éléments sont considérés comme les plus avantageux et sont présents habituellement à une concentration pondérale (exprimée par rapport au métal nickel ou cobalt de Raney) inférieure à 10 %, de préférence inférieure à 5 %.

Les catalyseurs de Raney comprennent souvent des traces de métaux présents dans l'alliage utilisé pour la préparation desdits catalyseurs. Ainsi, l'aluminium est notamment présent dans ces catalyseurs.

Selon une caractéristique préférentielle de l'invention, la solution aqueuse basique utilisée pour traiter le catalyseur est une solution d'une base alcaline ou de l'ammoniaque. De préférence, le composé basique présente une solubilité dans l'eau élevée.

A titre de composés basiques convenables, on peut citer la soude, la potasse, l'hydroxyde de lithium ou de césium.

Avantageusement, la concentration en anions de cette solution basique est comprise entre 0,01 N et 10 N, de préférence comprise entre 0,02 N et 7 N.

La concentration pondérale du catalyseur dans la solution basique est comprise entre 5 et 30 % par rapport au milieu réactionnel.

Dans les variantes comprenant un traitement par l'hydrogène, le milieu réactionnel est soit constitué de la solution basique et du catalyseur tel que décrit précédemment, soit du catalyseur usé seul, ou du catalyseur traité par la solution basique et éventuellement lavé. Le réacteur est mis sous une pression partielle d'hydrogène, avantageusement supérieure à $10^5$ Pa.

Le milieu réactionnel est ensuite chauffé à une température inférieure à 130°C, de préférence comprise entre 100°C et 130°C pendant une durée déterminée par l'expérience et un étalonnage préalable du procédé. Cette durée est de l'ordre de quelques heures, avantageusement comprise entre 1 heure et 10 heures.

Pour permettre un échange avec l'hydrogène contenu dans le réacteur, le milieu réactionnel est avantageusement agité. Bien entendu, d'autres procédés d'alimentation de l'hydrogène, tels que barbotage, colonne à remplissage pourraient être utilisés pour la réalisation de ce traitement sans sortir de l'invention. Toutefois, un des avantages du procédé de l'invention est de permettre d'obtenir un traitement efficace avec un rendement de régénération élevé sans nécessité des procédés complexes pour favoriser le contact de l'hydrogène et du catalyseur.

Après avoir été traité par une solution basique et éventuellement subi une hydrogénation, le catalyseur est soit extrait du milieu soit concentré dans ce milieu par les techniques habituelles de concentration ou séparation telles que, par exemple, filtration, décantation, centrifugation, évaporation ou analogues. Le catalyseur ainsi séparé ou concentré est soumis à une étape de lavage pour éliminer partiellement la solution basique et également toutes les impuretés et composés qui limitent l'activité du catalyseur.

Ce lavage peut être effectué soit par de l'eau soit par une solution basique diluée. En effet, le lavage permet d'éliminer toutes les impuretés mais doit maintenir le pH du catalyseur à une valeur supérieure à 12, avantageusement compris entre 12 et 13. Ce pH est contrôlé par la mesure de celui des eaux de lavages.

Ainsi, dans un mode de réalisation préféré, notamment quand le procédé de régénération est réalisé en continu, la solution de lavage est une solution basique de concentration comprise entre 0,01 N et 0,1 N.

Le catalyseur ainsi régénéré peut être utilisé soit seul, soit en mélange avec un catalyseur neuf dans les procédés d'hydrogénation de composés comprenant des fonctions nitriles.

L'invention a également pour objet un procédé d'hydrogénation de composés comprenant au moins une fonction nitrile en composés aminés tel que décrit, par exemple, dans la demande de brevet WO 95/19 959.

Ce procédé consiste brièvement à ajouter le composé nitrile dans un milieu réactionnel selon une concentration pondérale comprise entre 0,001 % et 30 % par rapport au poids total du milieu réactionnel.

Le milieu réactionnel est un milieu liquide qui contient au moins un solvant. Ce milieu réactionnel contient avantageusement de l'eau dans une quantité de préférence inférieure ou égale à 50 %, préférentiellement comprise entre 0,1 et 15 % en poids.

En complément à l'eau, un solvant du type alcool et/ou amide peut être prévu tels que le méthanol, l'éthanol, le propanol, l'isopropanol, le butanol, les glycols, des polyols, le diméthylformamide ou le diméthylacétamide. Ce solvant, quand l'eau est présente, représente deux à quatre parties en poids par partie d'eau présente.

Dans un mode de réalisation préféré, le milieu réactionnel comprend également l'amine dont la préparation est visée par le procédé d'hydrogénation. Ainsi, dans le cas de l'hydrogénation de l'adiponitrile en hexaméthylène diamine, le procédé est réalisé en présence d'hexaméthylène diamine.

La concentration pondérale en amine est comprise entre 50 % et 99 % en poids par rapport au poids total du solvant du milieu réactionnel.

Le milieu réactionnel comprend avantageusement un composé basique quand le catalyseur est du type Raney.

Cette base est présente à une concentration supérieure à 0,1 mol/kg de catalyseur, de préférence entre 0,1 et 10 mol/kg.

La réaction d'hydrogénation est réalisée en maintenant dans l'enceinte une pression en hydrogène convenable c'est-à-dire comprise avantageusement entre 0,10 et 10 MPa, et une température du milieu réactionnel inférieure à 150°C, de préférence inférieure ou égale à 100°C.

Le procédé ci-dessus est donné à titre d'illustration. L'invention peut également s'appliquer à d'autres procédés dans lesquels l'hydrogénation est réalisée en présence d'ammoniac par exemple.

L'invention a également pour objet un procédé d'hémihydrogénation de composés comprenant au moins deux fonctions nitriles dans lesquels au moins une fonction nitrile est réduite en fonction amine.

De tels procédés sont notamment utilisés pour la synthèse de composés aminonitriles qui par hydrolyse cyclisante donnent des lactames. Ces lactames sont des monomères pour la fabrication d'homopolyamides.

Ainsi, une des applications industrialisées est la fabrication du caprolactame, monomère du polyamide 6, obtenu par hémihydrogénation de l'adiponitrile en aminocapronitrile et hydrolyse cyclisante de ce composé en caprolactame.

Ce procédé est notamment décrit dans les demandes de brevet WO 93/16 034, WO 93/12 073, US 4 248 799. Ce procédé utilise comme catalyseur un Nickel de Raney.

Ainsi, dans ce procédé, l'adiponitrile est ajouté dans un milieu réactionnel comprenant une base alcaline et/ou de l'ammoniaque, de l'hydrogène, un catalyseur Nickel de Raney et un complexe d'un métal de transition. Le milieu réactionnel peut contenir un solvant tel qu'un alcool.

Le catalyseur est soit dispersé dans le milieu réactionnel, soit supporté sur un lit fixe.

Dans un autre mode de réalisation le catalyseur de Raney (nickel ou cobalt) peut être traité avec un alcanolate alcalin, la réaction d'hydrogénation pouvant être réalisée en absence d'eau et en présence d'un solvant aprotique tel que le tétrahydrofuranne, la dioxane, les diamines aliphatiques, les alcools ou les éthers.

Le procédé d'hémihydrogénation peut également être réalisé en utilisant un catalyseur de Raney tel que le Nickel de Raney comprenant un élément dopant tel que défini précédemment.

Dans ce procédé, le milieu d'hydrogénation comprend de l'eau à raison d'au moins 0,5 % en poids par rapport à la totalité des composés liquides du milieu réactionnel, de l'aminonitrile et/ou de la diamine formées par l'hydrogénation, et du composé nitrile non transformé. Le milieu réactionnel comprend également une base minérale forte, à savoir un hydroxyde alcalin présent à une concentration de 0,1 mol/kg à 3 mol/kg de catalyseur.

Dans ces procédés, les catalyseurs peuvent être constitués par un mélange de catalyseur neuf et de catalyseur régénéré selon le procédé de l'invention. On peut également utiliser uniquement un catalyseur régénéré.

Les composés nitriles pouvant être réduits totalement ou partiellement par les procédés de l'invention sont, à titre d'exemple, les composés alpha - oméga dinitriles comportant une chaîne aliphatique linéaire ou ramifiée de 1 à 12 atomes de carbone tels que l'adiponitrile, le méthylglutaronitrile, l'éthylsuccinonitrile, le malononitrile, le succinonitrile, le glutaronitrile ou un mélange de ces composés.

De manière générale, les catalyseurs pouvant être régénérés par le procédé de l'invention sont avantageusement utilisés en association avec un composé basique dans les procédés d'hydrogénation des composés nitriles.

D'autres avantages, détails de l'invention apparaîtront plus clairement au vu des exemples donnés ci-dessous uniquement à titre indicatif sans effet limitatif.

**Exemple 1 :** Régénération d'un catalyseur Ni de Raney utilisé dans un procédé d'hydrogénation de l'adiponitrile (ADN) en hexaméthylène diamine (HMD)

L'hydrogénation de l'adiponitrile (AdN) est réalisée à l'aide d'un catalyseur à base de Nickel de Raney 50 (dopé à 1,8 % de Cr et 1 % de Fe) en présence d'un hydroxyde alcalin tel que KOH et/ou NaOH, selon le procédé décrit dans le brevet WO 95/17 959.

Quand l'activité du catalyseur n'est plus satisfaisante pour une exploitation correcte du procédé d'hydrogénation de l'adiponitrile, celui-ci est séparé du milieu réactionnel pour être régénéré selon le procédé de l'invention. Le catalyseur traité dans le présent exemple a une activité égale à 30% de l'activité du catalyseur neuf.

L'activité des catalyseurs est déterminée par le test catalytique standardisé suivant:

On prélève environ 1 à 2 g de bouillie de Ni Raney, que l'on lave 6 fois avec 50 ml d'eau distillée. On pèse exactement 0,40 g de catalyseur dans un pycnomètre. Le dit catalyseur est introduit dans un autoclave de 150 ml en acier inoxydable muni d'un système d'agitation, d'un système de chauffage, de moyens d'introduction d'hydrogène et de réactifs et de moyens de mesure et de contrôle de la température et de la pression. Avec le catalyseur, on entraîne aussi environ 0,4 g d'eau (cette quantité est prise en compte dans la composition pondérale des 42 g de solvant réactionnel composé de 90 % d'HMD et de 10 % d'eau). On charge dans l'autoclave sous atmosphère d'argon l'HMD, l'eau et de la potasse (à raison de 0,05% en poids du mélange réactionnel soit 0,8 mol KOH/kg Ni). L'autoclave est purgé à l'azote et à l'hydrogène. Il est ensuite chauffé à 80°C et maintenu sous 25 bar d'hydrogène par l'intermédiaire d'une réserve d'hydrogène. On met en route le système d'enregistrement de la pression d'hydrogène dans la dite réserve et on injecte rapidement 6 g d'AdN. L'hydrogénation est suivie jusqu'à la fin de la consommation d'hydrogène.

La vitesse initiale d'hydrogénation obtenue avec les catalyseurs usés et régénérés sont comparées à celle obtenue avec le Ni Raney 50 neuf, et l'activité d'un catalyseur est donnée par la formule suivante :

$$Activité \ du \ catalyseur = \frac{Vitesse \ initiale \ avec \ le \ catalyseur}{Vitesse \ initiale \ avec \ le \ Ni \ Raney \ 50 \ neuf} \times 100$$

Le catalyseur usé ci-dessus est mélangé avec une solution de soude 6N pour obtenir un mélange de composition massique suivante : Ni / NaOH 6N 18 / 82.

Ce mélange est chargé dans un ballon de 500 ml et agité pendant 3 h à reflux à 105 °C.

Le catalyseur est ensuite lavé à l'eau jusqu'à un pH égal à environ 12,5 pour éviter la précipitation des aluminates de sodium sous forme de trihydroxydes d'aluminium, pour éviter une désactivation du catalyseur pendant le stockage. Le pH est contrôlé par mesure du pH des eaux de lavage.

L'activité du catalyseur pour la fabrication de HMD qui était de 30% est égale après régénération, à 90%.

**Exemple 2** : Régénération d'un catalyseur Ni de Raney utilisé dans un procédé d'hémihydrogénation de l'Adiponitrile (ADN) en aminocapronitrile (ACN).

Le catalyseur utilisé dans un procédé d'hémihydrogénation de l'adiponitrile en aminocapronitrile réalisée selon les conditions décrites dans le brevet WO 93/16 034 est un Nickel de Raney identique à celui décrit dans l'exemple 1.

Le catalyseur usé récupéré présente une activité égale à 10% de celle d'un catalyseur neuf.

Ce catalyseur est mélangé avec une solution de soude 0,05N pour obtenir un rapport pondéral Catalyseur usé / phase liquide égal à 20/80.

Ce mélange est introduit dans un autoclave en acier inoxydable de 1,3 l équipé de façon similaire à l'appareil de 150 ml décrit précédemment.

Le réacteur est pressurisé à 20 bar avec de l'hydrogène, agité à 500 tr/min et chauffé à 120°C. Un transfert gaz/liquide élevé n'est pas nécessaire étant donné que la solubilité de l'hydrogène dans les conditions de régénération est suffisante. Après 1 h à 120°C, le réacteur est refroidi rapidement sous hydrogène et le mélange liquide/solide soutiré.

Le catalyseur est ensuite lavé en continu par une solution de NaOH 0,05 N.

L'activité du catalyseur avant régénération est de 10 %, après régénération cette activité est équivalente à celle du catalyseur neuf soit environ 100 %.

**Exemple 3 :** Régénération d'un catalyseur Ni de Raney utilisé dans un procédé d'hydrogénation de l'Adiponitrile (ADN) en hexaméthylène diamine (HMD)

Le catalyseur usé de l'exemple 1 a été soumis à une régénération selon le procédé décrit à l'exemple 2, la solution de soude ayant une concentration de 3N au lieu de 0,05N.

L'activité du catalyseur régénéré est équivalente à celle du catalyseur neuf.

**Exemple 4** : Régénération d'un catalyseur Ni de Raney utilisé dans un procédé d'hémihydrogénation de l'Adiponitrile (ADN) en aminocapronitrile (ACN) et hexaméthylène diamine

Les catalyseurs utilisés dans un procédé d'hydrogénation de l'adiponitrile en HMD et ACN tel que décrit dans le brevet US 5 151 543 sont régénérés selon le procédé décrit à l'exemple 2 ci-dessus.

Les catalyseurs usés provenant de la synthèse de l'ACN et de la synthèse de HMD dont l'activité est respectivement de 30% et 10% présentent, après régénération, une activité égale à celle des catalyseurs neufs.

## Revendications

**1.** - Procédé de régénération de catalyseurs de type catalyseur de Raney d'hydrogénation totale ou partielle des fonctions nitriles en fonctions amines de composés organiques, **caractérisé en ce qu'**il consiste à mélanger le catalyseur usé extrait du milieu réactionnel avec une solution aqueuse d'un composé basique présentant une concentration en ions basiques supérieure à 0,01 mol/l, à maintenir le mélange à une température inférieure à 130°C, puis à laver le catalyseur traité par de l'eau ou une solution basique avec un pH final des eaux de lavage compris entre 12 et 13.

**2.** - Procédé selon la revendication 1, **caractérisé en ce que** la solution aqueuse basique est une solution de base alcaline, de l'ammoniaque.

**3.** - Procédé selon la revendication 1, **caractérisé en ce que** la solution aqueuse basique est une solution de soude d'hydroxyde de lithium, d'hydroxyde de césium.

**4.** - Procédé selon l'une des revendications 1 à 3, **caractérisé en ce que** le lavage est effectué par une solution de soude de concentration comprise entre 0,01 N et 0,1N

**5.** - Procédé selon l'une des revendications 1 à 4, **caractérisé en ce que** le catalyseur usé, le catalyseur traité par une solution basique et éventuellement lavé, ou le mélange catalyseur usé/solution aqueuse basique avant ou après maintien à une température inférieure à 130°C, est mis sous atmosphère d'hydrogène et porté à une température inférieure à 130°C.

**6.** - Procédé selon la revendication 5, **caractérisé en ce que** le mélange contenant le catalyseur à traiter est agité.

**7.** - Procédé selon l'une des revendications précédentes, **caractérisé en ce que** le procédé de régénération est réalisé en continu, ou discontinu.

**8.** - Procédé selon l'une des revendications précédentes, **caractérisé en ce que** la concentration pondérale en catalyseur usé dans le mélange catalyseur/solution basique est comprise entre 5 % et 30 % par rapport au mélange.

**9.** - Procédé selon l'une des revendications 1 à 8, **caractérisé en ce que** le catalyseur de type métal de Raney est choisi dans le groupe comprenant le nickel de Raney, le cobalt de Raney.

**10.** - Procédé selon l'une des revendications 1 à 9, **caractérisé en ce que** le catalyseur de Raney comprend un élément dopant constitué par un élément des groupes IIB, IVB, IIIB, VB, VIB, VIIB et VIII de la table périodique de Mendeleev.

**11.** - Procédé selon la revendication 10, **caractérisé en ce que** l'élément dopant est choisi dans le groupe comprenant le titane, le chrome, le zirconium, le vanadium, le molybdène, le manganèse, le zinc, le tungstène, le fer.

**12.** - Procédé selon l'une des revendications précédentes, **caractérisé en ce que** le catalyseur est utilisé en association avec un composé basique dans les réactions d'hydrogénation.

**13.** - Procédé d'hydrogénation de composés comprenant au moins une fonction nitrile en composés aminés, **caractérisé en ce qu'**il utilise un catalyseur comprenant jusqu'à 100 % en poids de catalyseur régénéré selon l'une des revendications 1 à 12.

**14.** - Procédé d'hémihydrogénation de composés comprenant au moins deux fonctions nitriles en composés aminonitriles, **caractérisé en ce qu'**il utilise un catalyseur comprenant jusqu'à 100 % en poids de catalyseur régénéré selon l'une des revendications 5 à 12.

**15.** - Procédé selon la revendication 9 à 14, **caractérisé en ce que** le composé comprenant des fonctions nitriles est choisi dans le groupe comprenant les composés alpha - oméga dinitriles comportant une chaîne aliphatique linéaire ou ramifiée de 1 à 12 atomes de carbone tels que l'adiponitrile, le méthylglutaronitrile, l'éthylsuccinonitrile, le malononitrile, le succinonitrile, le glutaronitrile ou un mélange de ces composés.

**Claims**

**1.** Process for regenerating catalysts of the Raney catalyst type for the total or partial hydrogenation of nitrile functions into amine functions of organic compounds, **characterized in that** it consists in mixing the spent catalyst, extracted from the reaction medium, with an aqueous solution of a basic compound having a basic ion concentration of greater than 0.01 mol/l, in maintaining the mixture at a temperature below 130°C and then in washing the treated catalyst with water or a basic solution until the final pH of the washing waters is between 12 and 13.

**2.** Process according to Claim 1, **characterized in that** the basic aqueous solution is a solution of alkaline base or aqueous ammonia.

**3.** Process according to Claim 1, **characterized in that** the basic aqueous solution is a solution of sodium hydroxide, lithium hydroxide or caesium hydroxide.

**4.** Process according to one of Claims 1 to 3, **characterized in that** the washing is carried out with a sodium hydroxide solution having a concentration of between 0.01 N and 0.1 N.

**5.** Process according to one of Claims 1 to 4, **characterized in that** the spent catalyst, the catalyst treated with a basic solution and optionally washed, or the spent catalyst/basic aqueous solution mixture before or after maintenance at a temperature below 130°C, is placed under a hydrogen atmosphere and brought to a temperature below 130°C.

**6.** Process according to Claim 5, **characterized in that** the mixture containing the catalyst to be treated is stirred.

**7.** Process according to one of the preceding claims, **characterized in that** the regeneration process is carried out in continuous or batchwise mode.

**8.** Process according to one of the preceding claims, **characterized in that** the concentration by weight of spent catalyst in the catalyst/basic solution mixture is between 5% and 30% relative to the mixture.

**9.** Process according to one of Claims 1 to 8, **characterized in that** the catalyst of Raney metal type is chosen from the group comprising Raney nickel and Raney cobalt.

**10.** Process according to one of Claims 1 to 9, **characterized in that** the Raney catalyst comprises a dopant element consisting of an element from groups IIB, IVB, IIIB, VB, VIB, VIIB and VIII of the Periodic Table of the Elements.

**11.** Process according to Claim 10, **characterized in that** the dopant element is chosen from the group comprising titanium, chromium, zirconium, vanadium, molybdenum, manganese, zinc, tungsten and iron.

**12.** Process according to one of the preceding claims, **characterized in that** the catalyst is used in combination with a basic compound in the hydrogenation reactions.

**13.** Process for hydrogenating compounds comprising at least one nitrile function into amine compounds, **characterized in that** it uses a catalyst comprising up to 100% by weight of regenerated catalyst according to one of Claims 1 to 12.

**14.** Process for semi-hydrogenating compounds comprising at least two nitrile functions into aminonitrile compounds, **characterized in that** it uses a catalyst comprising up to 100% by weight of regenerated catalyst according to one of Claims 5 to 12.

15. Process according to Claims 9 to 14, **characterized in that** the compound comprising nitrile functions is chosen from the group comprising α,ω-dinitrile compounds comprising a linear or branched aliphatic chain of 1 to 12 carbon atoms, such as adiponitrile, methyl glutaronitrile, ethyl succinonitrile, malononitrile, succinonitrile, glutaronitrile or a mixture of these compounds.

**Patentansprüche**

1. - Verfahren zur Regenerierung von Katalysatoren vom Typ Raney-Katalysator für die vollständige oder teilweise Hydrierung von Nitrilfunktionen zu Aminofunktionen von organischen Verbindungen, **dadurch gekennzeichnet, dass** es darin besteht, den verbrauchten, aus dem Reaktionsmedium abgetrennten Katalysator mit einer wässrigen Lösung einer basischen Verbindung, die eine Konzentration an basischen Ionen höher als 0,01 mol/l aufweist, zu mischen, das Gemisch auf einer Temperatur unterhalb von 130 °C zu halten, dann den behandelten Katalysator mit Wasser oder einer basischen Lösung mit einem End-pH der Waschwässer zwischen 12 und 13 zu waschen.

2. - Verfahren gemäß Anspruch 1, **dadurch gekennzeichnet, dass** die basische wässrige Lösung eine Lösung einer Alkalibase oder von Ammoniak ist.

3. - Verfahren gemäß Anspruch 1, **dadurch gekennzeichnet, dass** die basische wässrige Lösung eine Lösung von Natriumhydroxid, von Lithiumhydroxid, von Cäsiumhydroxid ist.

4. - Verfahren gemäß einem der Ansprüche 1 bis 3, **dadurch gekennzeichnet, dass** das Waschen mit einer Natriumhydroxidlösung mit einer Konzentration zwischen 0,01 N und 0,1 N ausgeführt wird.

5. - Verfahren gemäß einem der Ansprüche 1 bis 4, **dadurch gekennzeichnet, dass** der verbrauchte Katalysator, der mit einer basischen Lösung behandelte und gegebenenfalls gewaschene Katalysator oder das Gemisch verbrauchter Katalysator/basische wässrige Lösung vor oder nach Halten auf einer Temperatur unterhalb von 130 °C unter Wasserstoffatmosphäre gesetzt und auf eine Temperatur unterhalb von 130 °C gebracht wird.

6. - Verfahren gemäß Anspruch 5, **dadurch gekennzeichnet, dass** das Gemisch, das den zu behandelnden Katalysator enthält, gerührt wird.

7. - Verfahren gemäß einem der vorhergehenden Ansprüche, **dadurch gekennzeichnet, dass** das Regenerierungsverfahren kontinuierlich oder diskontinuierlich durchgeführt wird.

8. - Verfahren gemäß einem der vorhergehenden Ansprüche, **dadurch gekennzeichnet, dass** die gewichtsbezogene Konzentration an verbrauchtem Katalysator in dem Gemisch Katalysator/basische Lösung zwischen 5 % und 30 %, bezogen auf das Gemisch, liegt.

9. - Verfahren gemäß einem der Ansprüche 1 bis 8, **dadurch gekennzeichnet, dass** der Katalysator vom Typ Raney-Metall ausgewählt ist aus der Gruppe, die Raney-Nickel, Raney-Kobalt umfasst.

10. - Verfahren gemäß einem der Ansprüche 1 bis 9, **dadurch gekennzeichnet, dass** der Raney-Katalysator ein Dotierungselement umfasst, das aus einem Element der Gruppen IIB, IVB, IIIB, VB, VIB, VIIB und VIII des Mendelejew-Periodensystems besteht.

11. - Verfahren gemäß Anspruch 10, **dadurch gekennzeichnet, dass** das Dotierungselement ausgewählt ist aus der Gruppe, die Titan, Chrom, Zirkonium, Vanadium, Molybdän, Mangan, Zink, Wolfram, Eisen umfasst.

12. - Verfahren gemäß einem der vorhergehenden Ansprüche, **dadurch gekennzeichnet, dass** der Katalysator in Kombination mit einer basischen Verbindung bei den Hydrierungsreaktionen verwendet wird.

13. - Verfahren zur Hydrierung von Verbindungen, die wenigstens eine Nitrilfunktion umfassen, zu Aminoverbindungen, **dadurch gekennzeichnet, dass** es einen Katalysator verwendet, der bis zu 100 Gew.-% gemäß einem der Ansprüche 1 bis 12 regenerierten Katalysator umfasst.

14. - Verfahren zur Hemihydrierung von Verbindungen, die wenigstens zwei Nitrilfunktionen umfassen, zu Aminonitrilverbindungen, **dadurch gekennzeichnet, dass** es einen Katalysator verwendet, der bis zu 100 Gew.-% gemäß einem der Ansprüche 5 bis 12 regenerierten Katalysator umfasst.

15. - Verfahren gemäß Anspruch 9 bis 14, **dadurch gekennzeichnet, dass** die Verbindung mit Nitrilfunktionen ausgewählt ist aus der Gruppe, die die alpha-omega-Dinitrilverbindungen mit einer linearen oder verzweigten aliphatischen Kette mit 1 bis 12 Kohlenstoffatomen, wie Adiponitril, Methylglutaronitril, Ethylsuccinonitril, Malononitril, Succinonitril, Glutaronitril oder ein Gemisch dieser Verbindungen, umfasst.